# EUROPEAN PATENT APPLICATION

(11) **EP 2 669 247 A1**
(43) Date of publication of application: **04.12.2013**
(21) Application number: 12170575.0
(22) Date of filing: 01.06.2012
(51) Int. Cl.: C01B 7/01, C07D 303/08, C07C 31/36, C07C 29/62

(54) **Process for manufacturing dichloropropanol**

(71) Applicant: SOLVAY SA, 1120 Bruxelles (BE)
(72) Inventor: Delannoy, Vincent, 7911 Oeudeghein (BE)
(74) Representative: Vande Gucht, Anne

(57) **Abstract**

Process for manufacturing dichloropropanol, comprising reacting glycerol with hydrogen chloride, wherein at least one part of the hydrogen chloride has been obtained by reacting hydrogen and chlorine, in a molar ratio between hydrogen and chlorine lower than or equal to 1.

## Description

The present invention relates to a process for manufacturing dichloropropanol.

The present invention relates more specifically to a process for manufacturing dichloropropanol from glycerol.

Dichloropropanol is a reaction intermediate in the preparation of epichlorohydrin and of epoxy resins (Kirk-Othmer Encyclopedia of Chemical Technology, Fourth Edition, 1992, Vol. 2, page 156, John Wiley & Sons, Inc.).

According to known processes it is possible to obtain dichloropropanol in particular by hypochlorinating allyl chloride, by chlorinating allyl alcohol and by hydrochlorinating glycerol. This latter process has the advantage that the dichloropropanol can be obtained starting from fossil raw materials or from renewable raw materials, and it is known that natural petrochemical resources, from which the fossil materials are obtained, such as petroleum, natural gas or coal, for example, are limited in their terrestrial availability.

DE 1075103 of VEB LEUNA - WERKE discloses the manufacture of glycerol dichlorohydrin by reaction of glycerol with hydrogen chloride, the hydrogen chloride being obtained through reaction of hydrogen with chlorine.

While such process appears to be attractive, opportunities for improvement remain, in particular, for the industrial implementation using such hazardous chemicals.

In a first embodiment, the present invention therefore relates to a process for manufacturing dichloropropanol, comprising reacting glycerol with hydrogen chloride, wherein at least one part of the hydrogen chloride has been obtained by reacting hydrogen and chlorine, in a molar ratio between hydrogen and chlorine lower than or equal to 1.

One of the essential features of the present invention lies in the molar ratio between hydrogen and chlorine lower than or equal to 1. It has surprisingly been found that an excess of chlorine in the produced hydrogen chloride is not detrimental to the production of dichloropropanol by reaction with glycerol.

Using a molar ratio between hydrogen and chlorine lower than or equal to 1 has the following non limiting advantages :
■ No residual hydrogen in hydrogen chloride and no risk of accumulation of explosive mixtures in parts of the dichloropropanol manufacturing plant ;
■ Possibility of using processes generating hydrogen and chlorine in equimolar amounts, like for example electrolysis of brine and/or hydrogen chloride, with
   o no import of extra hydrogen,
   o captive consumption of hydrogen an chlorine, and limited storage of hazardous compounds,
   o useful valorisation of hydrogen excess through steam generation for instance, if the molar ratio between hydrogen and chlorine is lower than 1.

In the process according to the invention, by dichloropropanol, one intends to denote anyone of 1,3-dichloropropane-2-ol, 2,3-dichloropropane-1-ol, and mixture thereof. Mixtures consisting essentially of 1,3-dichloropropane-2-ol and 2,3-dichloropropane-1-ol are preferred. In the mixture of 1,3-dichloropropane-2-ol and 2,3-dichloropropane-1-ol, the 1,3-dichloro-2-propanol content with respect to the total content of the two dichloropropanol isomers is preferably higher than or equal to 100 g/kg, yet preferably higher than or equal to 300 g/kg, still preferably higher than or equal to at least 400 g/kg, more preferably higher than or equal to 750 g/kg, still more preferably higher than or equal to 800 g/kg, yet more preferably higher than or equal to 900 g/kg, and most preferably higher than or equal to 920 g/kg. This content of 1,3-dichloro-2-propanol in the mixture is generally at most 990 g/kg and usually at most 960 g/kg. Contents of 925, 930, 935, 940, 945, 950 or 955 g/kg are particularly convenient. It is also possible to use a dichloropropanol composed essentially of 1,3-dichloro-2-propanol.

In the process according to the invention, the glycerol may be obtained from fossil or renewable raw materials. It is preferred to use glycerol obtained from renewable materials. A glycerol which is particularly suitable may be obtained during the conversions of fats or oils of vegetable or animal origin, such as saponification, transesterification or hydrolysis reactions. A particularly suitable glycerol may be obtained during the conversion of animal fats. Another particularly suitable glycerol may be obtained during the manufacture of biodiesel. Another particularly suitable glycerol may be obtained during the fatty acid manufacture. Another particularly suitable glycerol may be obtained during the fatty alcohols manufacture.

In the process according to the invention, the dichloropropanol has preferably been obtained by reacting hydrogen chloride with glycerol obtained from renewable raw materials.

In the process according to the invention, the part of the hydrogen chloride which has possibly not been obtained by reacting hydrogen and chlorine may come from any other sources, like for instance, from the manufacture of vinyl chloride, of mono- and polyisocyanates, preferably 4,4'-methylenediphenyl diisocyanate (MDI) or toluene diisocyanate (TDI) or hexamethylen-1,6-diisocyanate (HDI), of allyl chloride, of chloromethanes, of silica, e.g by flame hydrolysis of silicon tetrachloride, of chlorohydrofluorocarbons, e.g. by hydrodechlorination of chlorofluorcarbons, of hydrofluorocarbons, e.g. by hydrodechlorination of chlorofluorcarbons and/or chlorohydrofluorcarbons, or in the chlorinolysis of organic compounds, or by high temperature oxidation of chlorinated organic compounds.

In the process according to the invention, preferably at least 90 %, more preferably at least 95 % and most preferably at least 99 % of the hydrogen chloride reacted with the glycerol has been obtained by reacting hydrogen and chlorine in a molar ration between hydrogen and chlorine lower than or equal to 1. In the process according to the invention, preferably at most 99.99 %, of the hydrogen chloride reacted with the glycerol has been obtained by reacting hydrogen and chlorine in a molar ration between hydrogen and chlorine lower than or equal to 1. A process wherein substantially all of the hydrogen chloride reacted with the glycerol, has been obtained by reacting hydrogen and chlorine is particularly suitable.

In the process according to the invention, the molar ratio between hydrogen and chlorine is often lower than or equal to 0.99, frequently lower than or equal to 0.95, in particular lower than or equal to 0.90 and more specifically lower than or equal to 0.75. That molar ration is usually higher than or equal to 0.5.

In the process according to the invention, the hydrogen chloride is preferably gaseous hydrogen chloride or a mixture of gaseous hydrogen chloride and of an aqueous solution of hydrogen chloride.

In the process according to the invention, hydrogen and chlorine may be reacted in any suitable reactors. For instance, chlorine and hydrogen can be fed by separate concentric tubes into a combustion chamber where chlorine bums with in a flame at a temperature higher than 2000°C.

Silica is a good material of construction for the burner. Cast iron or steel, sometimes water-cooled, or graphite can also been used. The burner is usually located on the bottom of a cylindrical combustion chamber, with the flame directed vertically upward. The combustion chamber can made of steel lined with refractory brick. A cooling section of appropriate size and shape can be connected to the combustion chamber. If chlorine and hydrogen are very moist or otherwise contaminated, corrosion-resistant construction materials such as silica or graphite can be used.

In the process according to the invention, the hydrogen chloride may be used under any form, selected from the group consisting of gaseous hydrogen chloride, an aqueous solution of hydrogen chloride, and any combination thereof. The use of gaseous hydrogen chloride is preferred, and the use of a combination of gaseous hydrogen chloride and of an aqueous solution of hydrogen chloride is more preferred.

In the process according to the invention, at least one fraction of the chlorine has preferably been obtained by electrolysis of an aqueous composition containing chloride. Preferably at least 50 %, more preferably at least 90 %, still more preferably at least 95 % and most preferably at least 99 % of the chlorine has been obtained by electrolysis of an aqueous composition containing chloride. In the process according to the invention, it is convenient that substantially all of the chlorine has been obtained by electrolysis of an aqueous composition containing chloride.

In the process according to the invention, at least another fraction of the chlorine may have been obtained by a process selected from oxidation, preferably catalytic, of hydrogen chloride by oxygen, of hydrogen chloride by nitric acid, of metallic chlorides by nitric acid, and any combination thereof.

In the process according to the invention, and more specifically when the chlorine has been obtained by electrolysis of an aqueous composition containing chloride, the chlorine exhibits a purity which is usually higher than or equal to 95 % by weight, often higher than or equal to 99.5 % by weight, frequently higher than or equal to 99.9 % by weight, and in particular higher than or equal to 99.99 % by weight. The chlorine purity is usually lower than or equal to 99.9999 % by weight.

In the process according to the invention, at least one fraction of the hydrogen has preferably been obtained by electrolysis of an aqueous composition containing chloride. Preferably at least 50 %, more preferably at least 90 %, still more preferably at least 95 % and most preferably at least 99 % of the hydrogen has been obtained by electrolysis of an aqueous composition containing chloride.

In the process according to the invention, it is convenient that substantially all of the hydrogen has been obtained by electrolysis of an aqueous composition containing chloride.

In the process according to the invention, at least another fraction of the hydrogen may have been obtained by a process selected from coke oven gas, coal and hydrocarbons gaseification, catalytic reforming of hydrocarbons, refinery processes, petrochemical processes, electrolysis of water, thermochemical water cleavage, radiolytic water cleavage, photochemical water cleavage, photoelectrical water cleavage, biological systems, conversion of metals, from ammonia decomposition, from methanol decomposition, from hydrogen sulphide decomposition, and any combination thereof.

In the process according to the invention, and more specifically when the hydrogen has been obtained by electrolysis of an aqueous composition containing chloride exhibits a purity which is usually higher than or equal to 95 % by weight, often higher than or equal to 99.5 % by weight, frequently higher than or equal to 99.9 % by weight, and in particular higher than or equal to 99.99 % by weight. The hydrogen purity is usually lower than or equal to 99.9999 % by weight.

In the process according to the invention, at least one fraction of the chlorine and at least one fraction of the hydrogen have preferably been obtained by electrolysis of an aqueous composition containing chloride. Preferably at least 50 %, more preferably at least 90 %, still more preferably at least 95 % and most preferably at least 99 % of the chlorine and of the hydrogen have been obtained by electrolysis of an aqueous composition containing chloride. In the process according to the invention, it is convenient that substantially all of the chlorine and of the hydrogen have been obtained by electrolysis of an aqueous composition containing chloride.

In the process according to the invention, when a fraction of the chlorine, or of the hydrogen or of both the chlorine and the hydrogen has been obtained by electrolysis of an aqueous composition containing chloride, the aqueous composition containing chloride, may be an aqueous composition containing hydrogen chloride, an aqueous composition containing a metal salt, or any combination thereof. The aqueous composition containing chloride is preferably an aqueous composition containing a metal salt, and more preferably a brine containing sodium chloride. The content of sodium chloride in the brine is generally higher than or equal to 10 g per kg of brine, in many cases higher than or equal to 30 g/kg, usually higher than or equal to 70 g/kg, frequently higher than or equal to 100 g/kg, often higher than or equal to 140 g/kg, in particular higher than or equal to 160 g/kg and most specifically higher than or equal to 200 g/kg. The sodium chloride content is usually lower than or equal to 350 g/kg, commonly lower than or equal to 325 g/kg, generally lower than or equal to 270 g/kg, often lower than or equal to 250 g/kg, and frequently lower than or equal to 230 g/kg. A sodium chloride content of about 200 g/kg is particularly convenient.

In the process according to the invention, when a fraction of the chlorine, or of the hydrogen or of both the chlorine and the hydrogen has been obtained by electrolysis of a brine containing sodium chloride, the electrolysis may be carried out in a chlor-alkali electrolysis cell. The chlor-alkali electrolysis cell may be selected from a chlor-alkali mercury electrolysis cell, a chlor-alkali diaphragm electrolysis cell, a chlor-alkali membrane electrolysis cell, and any combination thereof. A a chlor-alkali membrane electrolysis cell is more preferred.

In the process according to the invention, the aqueous composition containing chloride is preferably a brine containing sodium chloride and the electrolysis is preferably carried out in a chlor-alkali membrane electrolysis cell.

In the process according to the invention, when a fraction of the chlorine, or of the hydrogen or of both the chlorine and the hydrogen has been obtained by electrolysis of a brine containing sodium chloride, the brine has preferably been obtained by reacting dichloropropanol, with a compound containing at least one active hydrogen atom and sodium hydroxide.

In that aspect of the process according to the invention, the dichloropropanol may have been obtained by reacting glycerol and hydrogen chloride or may be derived from any other processes such as, for example, the allyl chloride hypochlorination process, the allyl alcohol chlorination process, the glycerol monochlorohydrin hydrochlorination process, the epichlorohydrin hydrochlorination process, the 2,3-dichloropropionaldehyde hydrogenation process as described in documents WO 1997/48667, US 6,350,922 and US 5,744,655, the 1,2-dichlorethylene hydroformylation process as described in document WO 2005/116004, the 1,3-dichloroacetone hydrogenation process as described in documents WO 2005/097722 and WO 2003/064357. 2,3-dichloropropionaldehyde may itself be obtained by chlorination of acrolein and/or hydroformylation of 1,2-dichloroethylene as described in documents US 2,860,146 and WO 2005/116004. 1,3-dichloroacetone may itself be obtained by chlorination of acetone and/or by bromine/chlorine exchange starting from 1,3-dibromoacetone as described in Applications WO 2005/097722 and WO 2005/115954. Acrolein may be obtained by selective oxidation of propylene. 1,2-dichloroethylene may be a by-product of the synthesis of vinyl chloride starting from ethane and/or be obtained by chlorination of acetylene. Acetylene may be obtained by conventional processes such as hydrolysis of calcium carbide and/or pyrolysis of hydrocarbons, crude oil and even coal, such as described in "Industrial Organic Chemistry, Third, Completely Revised Edition, VCH, 1997, pp. 93-98". 1,3-dibromoacetone may be obtained by bromination of acetone, as described in document WO 2005/115954. Acetone may itself be obtained by conventional processes, such as, for example, oxidation of propylene, dehydrogenation of isopropanol and/or decomposition of cumene hydroperoxide, as described in "Industrial Organic Chemistry, Third, Completely Revised Edition, VCH, 1997, pp. 276-277 and 347-355".

In that variant, the dichloropropanol is preferably obtained by reaction between glycerol and a chlorinating agent as described in Patent Applications WO 2005/054167, WO 2006/100311, WO 2006/100312, WO 2006/100313, WO 2006/100314, WO 2006/100315, WO 2006/100316, WO 2006/100317, WO 2006/106153, WO 2007/054505, WO 2006/100318, WO 2006/100319, WO 2006/100320, WO 2006/106154, WO 2006/106155, WO 2007/144335, WO 2008/107468, WO 2008/101866, WO 2008/145729, WO 2008/110588, WO 2009/000773, WO 2009/043796, WO 2009/121853, WO 2009/077528, WO 2010/066660, WO 2010/029039 and WO 2010/029153, filed in the name of SOLVAY, the contents of which are incorporated herein by reference.

In that aspect of the process according to the invention, the compound containing at least one active hydrogen atom can be of any type. This compound may have been from renewable raw materials, fossil raw materials and any combination thereof. This compound is preferably obtained from renewable raw materials.

The compound containing at least one active hydrogen atom is preferably selected from the group consisting of a polyol, a polycarboxylic acid, a polyamine, an amino alcohol, a polyimide, a polyamide, a polyaminoamide, a polyimine, an acid mono- or polyphenol and mixtures of at least two of these compounds.

In the process according to the invention, the compound containing at least one active hydrogen atom is more often a polyol selected from the group constituted of bisphenol A (4,4'-dihydroxy-2,2-diphenylpropane, 4,4'-isopropylidenediphenol), tetrabromobisphenol A (4,4'-isopropylidenebis(2,6-dibromophenol)), bisphenol AF (4,4'-[2,2,2-trifluoro-1-(trifluoromethyl)ethylidene]bisphenol), hexafluorobisphenol A (4,4'-dihydroxy-2,2-diphenyl-1,1,1,3,3,3-hexafluoropropane), 1,1,2,2-tetra(p-hydroxyphenyl)ethane, tetramethylbisphenol (4,4'-dihydroxy-3,3',5,5'-tetramethyl bisphenol), 1,5-dihydroxynaphthalene, 1,1',7,7'-tetrahydroxydinaphthylmethane, 4,4'-dihydroxy-α-methylstilbene, a condensation product of bisphenol A with formaldehyde (bisphenol A novolac), a condensation product of phenol with formaldehyde, preferably bisphenol F (mixture of o,o', o,p' and p,p' isomers of dihydroxydiphenylmethane), a condensation product of cresol with formaldehyde (mixture of o,o', o,p' and p,p' isomers of methylhydroxydiphenylmethane), an alkylation product of phenol and of dicyclopentadiene (2,5-bis[hydroxyphenyl]octahydro-4,7-methano-5H-indene), a condensation product of phenol and of glyoxal (tetrakis(4-hydroxyphenyl)ethane), a condensation product of phenol and of a hydroxybenzaldehyde (e.g. tris(4-hydroxyphenyl)methane), 1,1,3-tris(p-hydroxyphenyl)propane, and mixtures of at least two of them, or p-aminophenol.

In the process according to the invention, the compound containing at least one active hydrogen atom is more frequently a polyol, preferably containing more than three carbon atoms, selected from the group consisting of a polyphenol, a sugar, a polyol derived from a sugar, an acid polyphenol, any derivative thereof, and any mixture thereof. The polyol may be as described in international application EP2011/066689 filed in the name of SOLVAY S.A., the content of which is incorporated herein by reference, more specifically the passages from page 12, line 5, to page 16, line 13.

In the process according to the invention, when the polyol is a product derived from a sugar, the product derived from a sugar is preferably selected from the group consisting of an anhydrosugar, a reduction product from sugar, a reduction product of hydroxymethylfurfural, a difuran derivative of furfural and any mixture thereof.

In the process according to the invention, when the product derived from a sugar is an anhydrosugar, it is preferably selected from the group consisting of isosorbide, isomannide, isoidide and any mixture thereof. The anhydrosugar is more preferably isosorbide.

In that aspect of the process according to the invention, the sodium hydroxide is usually used in the form an aqueous composition, preferably a concentrated aqueous solution or suspension, and more preferably in the form of a concentrated aqueous solution.

The content of sodium hydroxide in the composition is usually higher than or equal to 30 % by weight of composition, preferably higher than or equal to 32 % by weight, more preferably higher than or equal to 35 % by weight, and most preferably higher than or equal to 40 % by weight. The content of sodium hydroxide in the composition is usually lower than or equal to 70 % by weight of composition, preferably lower than or equal to 60 % by weight, and most preferably lower than or equal to 50 % by weight.

The dichloropropanol manufactured in the process according to the invention can be reacted with a compound containing at least one active hydrogen atom and sodium hydroxide in order to produced an epoxy resin and a brine containing sodium chloride, said brine can be electrolyzed in a chlor-alkali membrane electrolysis cell, in order to produce hydrogen, chlorine and sodium hydroxide, at least one fraction of said hydrogen, chlorine and sodium hydroxide can be recycled in the process for manufacturing the epoxy resin.

Therefore, in as second embodiment, the present invention relates to a process for a process for manufacturing an epoxy resin, comprising the process for manufacturing the dichloropropanol by reacting glycerol with hydrogen chloride, wherein at least one part of the hydrogen chloride has been obtained by reacting hydrogen and chlorine, in a molar ratio between hydrogen and chlorine lower than or equal to 1, and reacting the dichloropropanol obtained with a compound containing at least one active hydrogen atom and sodium hydroxide, wherein a brine containing sodium chloride is generated, said brine is electrolyzed in a chlor-alkali membrane electrolysis cell, in order to produce hydrogen, chlorine and sodium hydroxide, and at least one fraction of said hydrogen, chlorine and sodium hydroxide is recycled in the process for manufacturing the epoxy resin.

In the process according to the invention, the expression "epoxy resin" is understood to mean a monomer or a polymer, the chemical formula of which contains at least one 2,3-epoxypropyloxy group. The epoxy resin is preferably a polymer.

Examples of monomers are for instance, monoglycidyl ethers, polyglycidyl ethers, or a mixture thereof. The polyglycidyl ether can be a diglycidyl ether, like the diglycidyl ether of bisphenol A (DGEBA) or the diglycidyl ether of isosorbide (diglycidylether of 1,4:3,6-dianhydro-D-sorbitol) for instance.

The term "polymer" is understood to mean molecules comprising several units joined to one another by covalent bonds, often in a repeating manner, these units being referred to as repeating units. The number of repeating units is greater than zero. A polymer contains at least one type of repeating unit. When the polymer contains only a single type of repeating unit, it is known as a homopolymer. When the polymer contains more than a single type of repeating unit, it is known as a copolymer. The copolymer may be of statistical, alternating or block type, as described in "Polymer Science Dictionary, M.S.M., Elsevier Applied Science, London and New York, 1989, page 86".

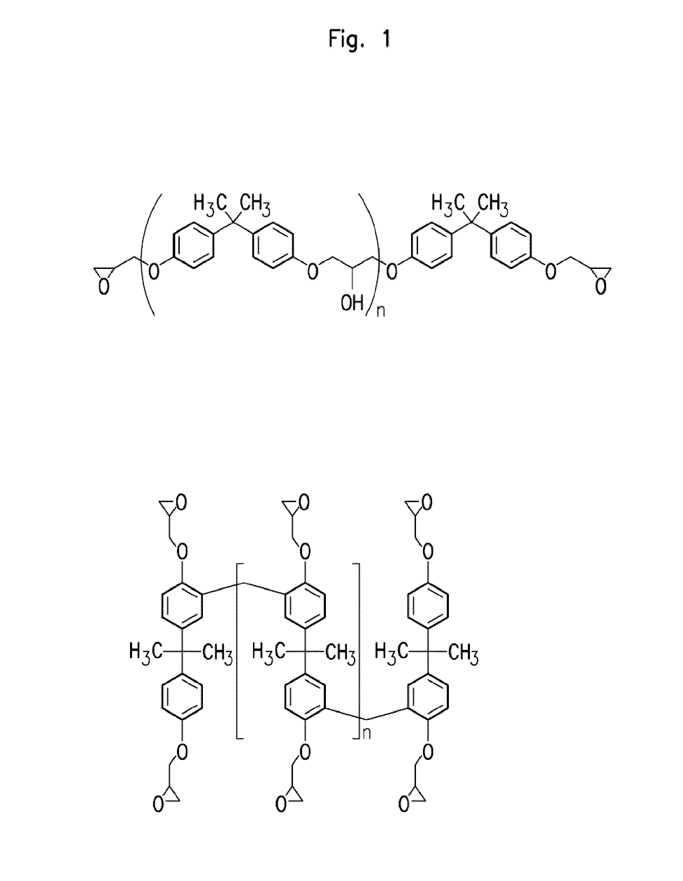

Examples of chemical formulae of epoxy resins are presented in Figure 1, when n is higher than or equal to zero and preferably higher than zero.

In the process according to the invention, the epoxy resin is preferably selected from the group consisting of Type I Grade 1 Classes A to H, Type II Grade 1 Classes A to F and Type VI Grade 1 Class A resins, as defined in the ASTM D 1763 - 00 (2005) standard entitled "Standard Specifications for Epoxy Resins", and any mixture thereof.

In the process according to the invention, the epoxy resin is more preferably a liquid epoxy resin. The expression "liquid epoxy resin" is understood to mean Type I Grade 1 Classes A and B, Type II Grade 1 Classes A, B and C, Type IV Grade 1 Classes A to D, Type V Grade 1 Classes A and B and Type VI Grade 1 Class A resins, as defined in the ASTM D 1763 - 00 (2005) standard entitled "Standard Specifications for Epoxy Resins".

The process for making the epoxy resins can be of any type generating a brine containing sodium chloride such as described in International application WO 2008/152044 of SOLVAY, the content of which is incorporated here by reference especially the passage from page 17, line 23, to page 22, line 19.

In the process for making the epoxy resin according to the invention, the hydrogen and the chlorine obtained by electrolysis are preferably reacted in a molar ratio between hydrogen and chlorine lower than or equal to 1 in order to produce the hydrogen chloride, and at least one portion of the hydrogen chloride is reacted with glycerol in order to obtain the dichloropropanol.

In the process for making the epoxy resin according to the invention, the molar ratio between the hydrogen chloride consumed in the process for manufacturing the epoxy resin, and the sodium chloride generated in the said process, expressed as elemental chlorine (Cl) is higher than or equal to 0.9.

In the process for making the epoxy resin according to the invention, epichlorohydrin can be formed in situ during the reaction of the dichloropropanol with a compound containing at least one active hydrogen atom and sodium hydroxide. The process preferably comprises recovering at least one part of the epichlorohydrin and recycling at least one fraction of the recovered epichlorohydrin to the said reaction.

The process for making the epoxy resin according to the invention comprises preferably at least one liquid-liquid phase separation step to remove at least one part of the epichlorohydrin formed in situ.

In the process for making the epoxy resin according to the invention, at least one of the dichloropropanol, of the compound containing at least one active hydrogen atom, and of the basic agent, has preferably been obtained from renewable raw materials.

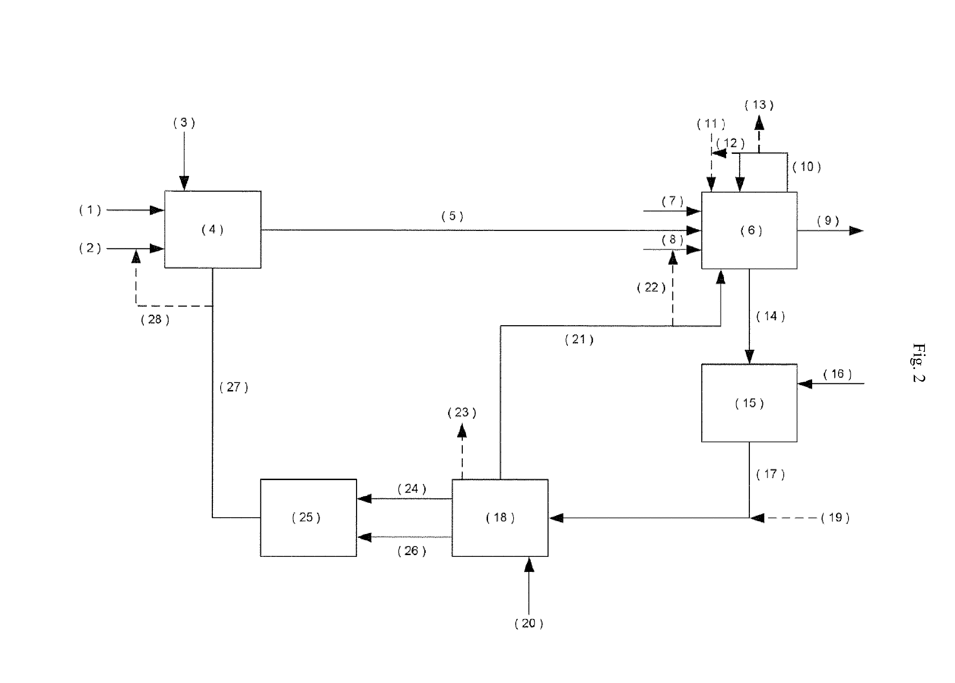

Figure 2 represents a non-limiting embodiment of the process according to the present invention.

A first stream of glycerol, preferably obtained from renewable raw materials, is fed to a first vessel (4) via a first line (1). A second stream of hydrogen chloride, gaseous, as an aqueous solution or a mixture thereof, is fed to first vessel (4) via a second line (2). A third stream of a catalyst, preferably a carboxylic acid is fed to first vessel (4) via a third line (3). First vessel (4) may comprise any combination of reactors and separation equipment. A fourth stream of dichloropropanol is withdrawn from first vessel (4) via a fourth line (5) and is fed to a second vessel (6). Also introduced in second vessel (6) are a fifth stream of a compound containing at least one active hydrogen atom via a fifth line (7) and a sixth stream of sodium hydroxide via a sixth line (8). A seventh stream of epoxy resin is withdrawn from second vessel (6) via a seventh line (9) which may be sent to storage, to further processing such as purification, or to other equipment for further reaction. An eight stream of epichlorohydrin formed in situ in second vessel (6) is withdrawn from second vessel (6) and recycled to second vessel (6) via an eighth line (10). A ninth stream of epichlorohydrin not formed in second vessel (6) may also be fed to second vessel (6) via a ninth line (11). Part of the eighth stream of epichlorohydrin may be added as a tenth stream to the ninth stream of epichlorohydrin via a tenth line (12). Part of the eight stream of the recycled epichlorohydrin may be withdrawn as an eleventh stream via a eleventh line (13) and may be sent to storage, to further processing such as purification, or to other equipment for further reaction. A twelfth stream of brine is withdrawn from second vessel (6) via a twelfth line (14) and fed to a third vessel (15). Third vessel (15) is also fed with a thirtheenth stream containing an oxidant, preferably "active chlorine" via a thirtheenth line (16). The expression "active chlorine" is understood to mean molecular chlorine and its reaction products with water, chloride ions or with a basic agent, such as hypochlorous acid, trichloride ion and sodium hypochlorite for example. A fourtheenth stream of brine is withdrawn from third vessel (16) via a fourtheenth line (17) and fed to a fourth vessel (18). A fifteenth stream of brine may be supplied via a fifteenth line (19). Also supplied to fourth vessel (18) is a sixteenth stream of electricity via a sixteenth line (20). A seventeenth stream of sodium hydroxide is withdrawn from fourth vessel (18) via a seventeenth line (21) and fed to second vessel (6). Part of the seventeenth stream of sodium hydroxide may be added as an eighteenth stream to the sixth stream of sodium hydroxide via a eighteenth line (22). A part of the seventeenth stream of sodium hydroxide may be withdrawn as a nineteenth stream via a nineteenth line (23). A twentieth stream of hydrogen is withdrawn from fourth vessel (18) via a twentieth line (24) and fed to a fifth vessel (25). Also withdrawn from fourth vessel (18) and fed to fifth vessel (25) is a twenty-first stream of chlorine via an twenty-first line (26). A twenty-second stream of hydrogen chloride is withdrawn from fifth vessel (25) and fed to first vessel (4) via a twenty-second line (27). Part of the twenty-second stream of hydrogen chloride may be added to the second stream of hydrogen chloride as a twenty-third stream via a twenty-third line (28).

Vessels (4), (6), (15), (18) and (25) contain reaction vessel of any well-known suitable type, including for example, one or more discontinuous or continuous reactors, and separation vessel of any well-known suitable type, including for example, one or more distillation columns, evaporators, strippers, settlers, adsorbers, absorbers, ion exchange equipments or combination thereof.

More specifically, vessel (18) contains any type of well-known electrolysis suitable cells, including for example, mercury, diaphragm and membrane chlor-alkali electrolysis cells.

## Claims

1. Process for manufacturing dichloropropanol, comprising reacting glycerol with hydrogen chloride, wherein at least one part of the hydrogen chloride has been obtained by reacting hydrogen and chlorine, in a molar ratio between hydrogen and chlorine lower than or equal to 1.

2. Process according to claim 1, wherein at least 90 % of the hydrogen chloride reacted with the glycerol has been obtained by reacting hydrogen and chlorine in said molar ratio.

3. Process according to claim 1 or 2, wherein the molar ratio between hydrogen and chlorine is lower than or equal to 0.9.

4. Process according to any one of claim 1 to 3, wherein the hydrogen chloride is gaseous hydrogen chloride or a mixture of gaseous hydrogen chloride and of an aqueous solution of hydrogen chloride.

5. Process according to any one of claims 1 to 4, wherein at least one fraction of the hydrogen and at least one fraction of the chlorine have been obtained by electrolysis of an aqueous composition containing chloride.

6. Process according to claim 5 wherein the aqueous composition containing chloride is a brine containing sodium chloride and wherein the electrolysis is carried out in a chlor-alkali membrane electrolysis cell.

7. Process according to claim 6, wherein the brine has been obtained by reacting dichloropropanol with a compound containing at least one active hydrogen atom and with sodium hydroxide.

8. Process for manufacturing an epoxy resin, comprising the process for manufacturing the dichloropropanol of any one of claims 1 to 7, and reacting the dichloropropanol obtained with a compound containing at least one active hydrogen atom and with sodium hydroxide, wherein a brine containing sodium chloride is generated, said brine is electrolyzed in a chlor-alkali membrane electrolysis cell, in order to produce hydrogen, chlorine and sodium hydroxide, and at least one fraction of said hydrogen, chlorine and sodium hydroxide is recycled in the process for manufacturing the epoxy resin.

9. Process according to claim 8, wherein the hydrogen and the chlorine obtained by electrolysis are reacted in a molar ratio between hydrogen and chlorine lower than or equal to 1 in order to produce hydrogen chloride, and at least one portion of the hydrogen chloride is reacted with glycerol in order to obtain the dichloropropanol.

10. Process according to any one of claims 7 to 9, wherein the molar ratio between the hydrogen chloride consumed in the process for manufacturing the epoxy resin, and the sodium chloride generated in the said process, expressed as elemental chlorine (Cl) is higher than or equal to 0.9.

11. Process according to any one of claims 7 to 10, wherein epichlorohydrin is formed in situ during the reaction of the dichloropropanol with a compound containing at least one active hydrogen atom and sodium hydroxide, preferably comprising recovering at least one part of the epichlorohydrin and recycling at least one fraction of the recovered epichlorohydrin to the said reaction.

12. Process according to claim 11 comprising at least one liquid-liquid phase separation step to remove at least one part of the epichlorohydrin formed in situ.

13. Process according to any one of claims 7 to 12 wherein the epoxy resin is selected from the group consisting of Type I Grade 1 Classes A to H, Type II Grade 1 Classes A to F and Type VI Grade 1 Class A resins, as defined in the ASTM D 1763 - 00 (2005) standard entitled "Standard Specifications for Epoxy Resins", and any mixture thereof, and wherein the epoxy resin is preferably a liquid epoxy resin selected from the group consisting of Type I Grade 1 Classes A and B, Type II Grade 1 Classes A, B and C and Type VI Grade 1 Class A resins, as defined in the ASTM D 1763 - 00 (2005) standard entitled "Standard Specifications for Epoxy Resins" and any mixture thereof.

14. Process according to any one of claims 7 to 13 wherein the compound containing at least one active hydrogen atom is a polyol selected from the group constituted of bisphenol A (4,4'-dihydroxy-2,2-diphenylpropane, 4,4'-isopropylidenediphenol), tetrabromobisphenol A (4,4'-isopropylidenebis(2,6-dibromophenol)), bisphenol AF (4,4'-[2,2,2-trifluoro-1-(trifluoromethyl)ethylidene]bisphenol), hexafluorobisphenol A (4,4'-dihydroxy-2,2-diphenyl-1,1,1,3,3,3-hexafluoropropane), 1,1,2,2-tetra(p-hydroxyphenyl)ethane, tetramethylbisphenol (4,4'-dihydroxy-3,3',5,5'-tetramethyl bisphenol), 1,5-dihydroxynaphthalene, 1,1',7,7'-tetrahydroxydinaphthylmethane, 4,4'-dihydroxy-α-methylstilbene, a condensation product of bisphenol A with formaldehyde (bisphenol A novolac), a condensation product of phenol with formaldehyde, preferably bisphenol F (mixture of o,o', o,p' and p,p' isomers of dihydroxydiphenylmethane), a condensation product of cresol with formaldehyde (mixture of o,o', o,p' and p,p' isomers of methylhydroxydiphenylmethane), an alkylation product of phenol and of dicyclopentadiene (2,5-bis[hydroxyphenyl]octahydro-4,7-methano-5H-indene), a condensation product of phenol and of glyoxal (tetrakis(4-hydroxyphenyl)ethane), a condensation product of phenol and of a hydroxybenzaldehyde (e.g. tris(4-hydroxyphenyl)methane), 1,1,3-tris(p-hydroxyphenyl)propane, and mixtures of at least two of them, or p-aminophenol or wherein the compound containing at least one active hydrogen atom is a polyol selected from the group consisting of a polyphenol, a sugar, a polyol derived from a sugar, preferably isosorbide, isomannide, isoidide and any mixture thereof, an acid polyphenol, any derivative thereof, and any mixture thereof.

15. Process according to any one of claims 7 to 14, wherein at least one of the dichloropropanol, of the compound containing at least one active hydrogen atom, and of the basic agent, has been obtained from renewable raw materials.
